# EUROPEAN PATENT APPLICATION

(11) **EP 4 837 502 A1**
(43) Date of publication of application: **11.11.2026**
(21) Application number: 25736426.5
(22) Date of filing: 03.01.2025
(51) Int. Cl.: A61B 5/053

(54) **BIOIMPEDANCE MEASUREMENT METHOD AND SYSTEM BASED ON MULTI-CHANNEL RADIO FREQUENCY TRANSMISSION**

(30) Priority: 03.01.2024 CN 202410007975
(71) Applicant: Brattea Medtech Co., Ltd., Shanghai 201114 (CN)
(72) Inventor: GUO, Jiulin, Shanghai 200231 (CN); HU, Hongyu, Shanghai 200231 (CN); WANG, Yan, Shanghai 200231 (CN)
(74) Representative: Wang, Bo
(86) International application number: PCT/CN2025/070621
(87) International publication number: WO 2025/146187

(57) **Abstract**

A bioimpedance measurement method and system based on multi-channel radio frequency transmission. The bioimpedance measurement method comprises the following steps: setting measurement-related parameters; a channel radio frequency control module switches from a radio frequency mode to a measurement mode; opening a measurement channel unit, adjusting the DCP value of the channel to a set second DCP value, closing the remaining channel units, and adjusting the DCP value of the closed channels to 0; the measurement channel unit emits a radio frequency signal, and analog DC voltage and analog DC current are obtained after conversion via circuits; an analog-to-digital conversion module collects the analog DC voltage and the analog DC current a set number of times and converts same into corresponding digital signals; and according to an impedance fitting formula, performing a calculation to obtain the impedance value of a current measurement by the channel.

## Description

### BACKGROUND

### Technical Field

The present invention belongs to the field of diagnosis and measurement technologies, and relates to a bioimpedance measurement method based on multi-channel radio frequency (RF) transmission, and also a corresponding bioimpedance measurement system.

### Related Art

Bioimpedance measurement is a detection technology for extracting, by using electrical characteristics of a biological tissue and an organ and variation patterns thereof, biomedical information related to a physiological condition and a pathological condition in the human body. Generally, in the technology, a tiny alternating measurement current or voltage is applied to a detection object by means of an electrode system placed on a body surface to detect corresponding electrical impedance and a change condition thereof. As one of the important parameters reflecting a physiological situation of a human body, the bioimpedance is an important indicator needing to be detected by many physiological parameter detection devices. Therefore, accuracy of the bioimpedance measurement is very important.

In the existing technology, a bioimpedance measurement method mainly includes a hardware impedance converter method and a software data fitting method. The implementation of the hardware impedance converter method is relatively complex. In the method, a detection system is formed through a DDS (signal generation) module, a DAC module, a GAIN (amplification) module, an ADC module, an FFT (Fourier calculation) module, a filter module, and the like. The DDS module is configured to transmit a signal of a fixed frequency, which reaches a to-be-detected feature tissue through the DDS module. After an adjustment through the GAIN module, the ADC module collects amplitudes of a voltage and a current, and after the amplitudes for a plurality of points are measured, a specific feature impedance value is calculated through the FFT module. However, when the method is applied to multi-channel radio frequency (RF) signal transmission, because a model for multi-channel RF signals in human body tissues is complex, complexity of signal discrimination increases exponentially. Even if a high-frequency impedance converter is used, a module distinguishing circuit is still required for distinguishing a DDS signal from an RF signal. Therefore, the method is not applicable to a multi-channel RF transmission method. In the software data fitting method, a large amount of collected data of a voltage, a current, and real impedance, then a linear relationship for the voltage, the current, and the impedance is found, and the linear relationship and a voltage value and a current value that are collected in real time are used to deduce a real impedance value during RF signal transmission. The method can be implemented by using relatively simple structures such as a voltage conversion module, a current conversion module, and an ADC module. However, as a power of an RF signal increases, when the RF signal is no longer a standard sine wave, a measurement error of the RF signal is large. Therefore, accurate measurement of bioimpedance is greatly affected.

### SUMMARY

A primary technical problem to be resolved in the present invention is to provide a bioimpedance measurement method based on multi-channel radio frequency (RF) transmission.

Another technical problem to be resolved in the present invention is to provide a bioimpedance measurement system based on multi-channel RF transmission.

To achieve the above objectives, the present invention adopts the following technical solutions:
According to a first aspect of embodiments of the present invention, a bioimpedance measurement method based on multi-channel RF transmission is provided, including the following steps:
(1) setting measurement-related parameters, including at least a number of channel units used during a current procedure, a number of times a voltage and a current are collected, and a second digitally controlled potentiometer (DCP) value of a channel;
(2) determining whether an impedance measurement command is received; and performing a next step if the measurement command is received;
(3) switching a channel RF control module from an RF mode to a measurement mode, and simultaneously recording a first DCP value of each channel;
(4) opening a measurement channel unit, and adjusting a DCP value of the channel to the set second DCP value; and simultaneously closing remaining channel units, and adjusting DCP values of closed channels to 0;
(5) emitting, by the measurement channel unit, an RF signal, and obtaining an analog direct current (DC) voltage and an analog DC current after conversion through a circuit; and collecting, by an analog-to-digital conversion module, the analog DC voltage and the analog DC current a set number of times, converting the collected analog DC voltage and the collected analog DC current into corresponding digital signals, and sending the corresponding digital signals to a microcontroller module;
(6) performing, by the microcontroller module, calculation according to an impedance fitting formula, to obtain an impedance value for a current measurement of the channel, and storing the impedance value in a buffer of a corresponding channel inside the microcontroller module; and incrementing a counter value by 1;
(7) determining whether a count value of a counter is equal to a set number of channel units; performing step (4) to perform measurement on a next channel unit when the count value is less than the set number of channel units; and performing a next step when the count value is equal to the set number of channel units;
(8) calculating, based on a plurality of measured impedance values of the channels, a current average impedance value of each of the channels, and displaying the average impedance value on a screen by a display; and resetting the counter;
(9) ending the current impedance measurement, switching the channel RF control module from the measurement mode to the RF mode, and restoring the DCP value of each of the channels to the first DCP value;
(10) determining whether impedance measurement during the current procedure is ended; performing a next step if the measurement is ended; and performing step (2) if next measurement is to be performed; and
(11) ending the impedance measurement.

Preferably, in step (6), the impedance value val(x, y) for current measurement is calculated by using the following formula: $val \left(x, y\right) = p 00 + \left(p10∗x\right) + \left(p01∗y\right) + \left(p20∗{x}^{2}\right) + \left(p11∗x∗y\right) + \left(p30∗{x}^{3}\right) + \left(\begin{matrix}p 21 \\ ∗ {x}^{2} ∗ y\end{matrix}\right)$ where x is a direct impedance value for the current measurement; y is the first DCP value; and p00, p10, p01, p20, p11, p30, and p21 are all p coefficients.

Preferably, the direct impedance value x is calculated through the following formula: $x = Vz_avg / Iz_avg$ where Vz_avg is an average voltage value, and Iz_avg is an average current value.

Preferably, when the second DCP value is set to 20, values of the p coefficients are as follows: $p 00 = - 102.7 ; p 10 = 448.6 ; p 01 = 0.01481 ; p 20 = - 301.4 ; p 11 = - 0.03425 ; p 30 = 117.7 ;$ and p21 = 0.02582.

Preferably, in step (6), a uniform number of times the impedance value is stored is set in a buffer of each of the channels inside the microcontroller module, and the buffer of each of the channels updates the stored impedance values according to a first-in first-out principle when a number of times the impedance values are stored exceeds the set number of times.

According to a second aspect of embodiments of the present invention, a bioimpedance measurement system based on multi-channel RF transmission is provided, including a microcontroller module, an upper computer, and at least one channel unit, where
the upper computer is connected to the microcontroller module, and is configured to supervise and control a bioimpedance measurement process;
the microcontroller module is connected to each of the channel units, and is configured to control an operating state of each of the channel units, perform calculation based on a measurement result from the channel unit, and finally obtain an average impedance value; and
the channel unit is configured to generate and output RF energy and measure impedance of a biological tissue.

Preferably, each of the channel units includes a channel RF control module, a transformer, a current transformer, a voltage transformer, a first rectifier circuit, a second rectifier circuit, a first relay, and an analog-to-digital conversion module, where
an output terminal of the channel RF control module is connected to an input terminal of the transformer, an output terminal of the transformer is connected to an input terminal of the first relay through the current transformer and the voltage transformer, and an output terminal of the first relay is connected to an electrode in close contact with a biological tissue; and an output terminal of the current transformer and an output terminal of the voltage transformer are respectively connected to an input terminal of the analog-to-digital conversion module through the first rectifier circuit and the second rectifier circuit, an output terminal of the analog-to-digital conversion module is connected to an input terminal of the microcontroller module, a first output terminal of the microcontroller module is connected to a control terminal of the channel RF control module, and a second output terminal of the microcontroller module is connected to a control terminal of the first relay.

Preferably, the channel RF control module includes a signal generator, a signal amplifier, a digitally controlled potentiometer (DCP), a driver amplifier, and a second relay, where
an output terminal of the signal generator is connected to an input terminal of the signal amplifier, an output terminal of the signal amplifier is connected to an input terminal of the DCP, an output terminal of the DCP is connected to an input terminal of the driver amplifier, and an output terminal of the driver amplifier is connected to the transformer in the channel unit;
a first control signal terminal of the microcontroller module is connected to a control terminal of the DCP, and a second control signal terminal of the microcontroller module is connected to a control terminal of the second relay; and an input terminal of the second relay is connected to a power supply terminal, and an output terminal of the second relay is connected to a power supply terminal of the driver amplifier.

Compared with the existing technology, the bioimpedance measurement method based on multi-channel RF transmission provided in the present invention uses the microcontroller module to precisely control each of the channel units, so that, for measuring each of the channels, multiple times of collection are performed under the same stable RF signal. In addition, control, collection, and feedback for each of channels are configured independently, thereby avoiding mutual interference between channels and implementing accurate bioimpedance measurement. In addition, because the bioimpedance measurement system uses simple and technically mature functional modules, such as the transformer, the transformer, the analog-to-digital conversion module, and the microcontroller module, the bioimpedance measurement system offers beneficial effects such as good stability, low implementation costs, and high reliability.

### BRIEF DESCRIPTION OF THE DRAWINGS

FIG. 1 is a schematic diagram of a circuit of a bioimpedance measurement system based on multi-channel radio frequency (RF) transmission according to an embodiment of the present invention;
FIG. 2 is a schematic structural diagram of a channel RF control module according to an embodiment of the present invention; and
FIG. 3 is a flowchart of a bioimpedance measurement method based on multi-channel RF transmission according to an embodiment of the present invention.

### DETAILED DESCRIPTION

Technical content of the present invention is described in detail below with reference to the accompanying drawings and specific embodiments.

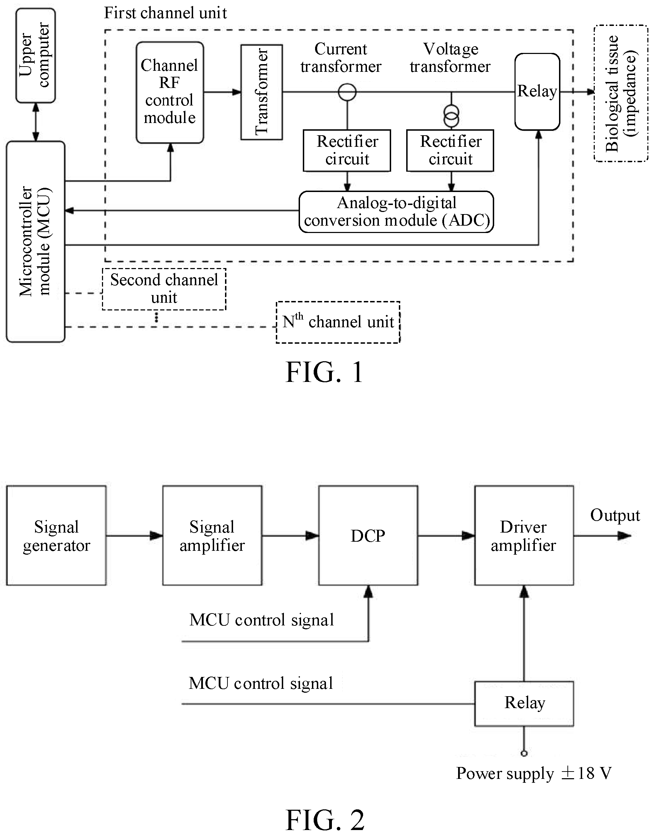

As shown in FIG. 1, a bioimpedance measurement system based on multi-channel radio frequency (RF) transmission provided in an embodiment of the present invention includes at least a microcontroller module (MCU for short), an upper computer, and at least one channel unit. Each of the channel units includes a channel RF control module, a transformer, a current transformer, a voltage transformer, a first rectifier circuit, a second rectifier circuit, a first relay, and an analog-to-digital conversion module (ADC for short).

The microcontroller module is configured to control an operating state of each of channel units and perform calculation based on a measurement result from the channel unit, to finally obtain an average impedance value. The upper computer is in communication with the microcontroller module and is configured to supervise and control a bioimpedance measurement process. The channel unit is configured to generate and output RF energy and measure impedance of a biological tissue. The structure of each of the channel units is the same, and a first channel unit is used as an example for structural description below.

A channel RF control module is configured to generate an RF signal and control a magnitude of an output RF signal and opening and closing of a channel. The structure thereof is shown in FIG. 2, including a signal generator, a signal amplifier, a digitally controlled potentiometer (DCP), a driver amplifier, and a second relay. The signal generator (DDS for short) is configured to generate a sine-wave RF signal of a particular frequency and amplitude; the signal amplifier is configured to amplify the RF signal; the DCP is configured to adjust the amplitude of the outputted RF signal based on an MCU control signal; the driver amplifier is configured to perform drive amplification on the RF signal; and the second relay is configured to control an on or off state of an operating power supply for the driver amplifier based on the MCU control signal.

In the channel RF control module, the magnitude of the RF signal outputted by the channel is dynamically adjusted by the MCU through the DCP controlled via an I2C bus, and a specific adjustment value of the DCP is recorded as a DCP value. An operating model for the channel RF control module is divided into an RF mode and a measurement mode. The RF mode is mainly used for providing energy for an intra-operation (for example, an ablation operation) to generate a heat effect for human tissues. A DCP value of a channel in the RF mode is recorded as a first DCP value. The measurement mode is mainly used for the impedance measurement of biological tissues, and the DCP value of the channel in the measurement mode is recorded as a second DCP value.

In an embodiment of the present invention, an X9C103 high-precision DCP is used as the DCP, and an output potential of the DCP is adjustable to 100 levels (also referred to as 100 steps or taps), that is, an adjustment range of a DCP value is 0 to 100.

The transformer is configured to perform step-down processing on an alternating current voltage outputted by the channel RF control module, so that a voltage outputted by a secondary side of the transformer satisfies measurement requirements.

The voltage transformer and the current transformer are configured to convert a voltage and a current outputted by the secondary side of the transformer, so that a secondary voltage outputted by the voltage transformer and a secondary current outputted by the current transformer satisfy measurement requirements for the analog-to-digital conversion module.

The first rectifier circuit and the second rectifier circuit are respectively configured to convert the secondary voltage outputted by the voltage transformer and the secondary current outputted by the current transformer into a DC voltage and a DC current.

The analog-to-digital conversion module is configured to measure the DC voltage and the DC current of a current circuit and convert them into digital signals that then are outputted to the microcontroller module.

The first relay is configured to control an on or off state of the channel unit based on a control signal of the MCU.

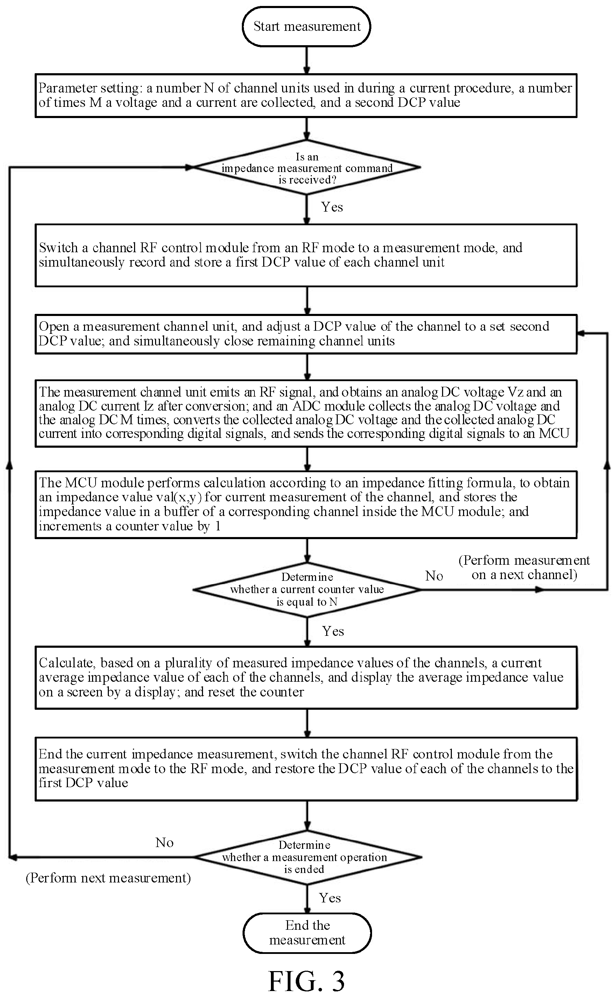

As shown in FIG. 3, a bioimpedance measurement method based on multi-channel RF transmission provided in an embodiment of the present invention includes the following steps:
S1: Set measurement-related parameters, where the parameters that need to be set include at least a number N of channel units used during a current procedure, a number M of times a voltage and a current are collected, and a second DCP value of a channel. N and M are both positive integers.

Usually, the second DCP value of the channel is a fixed value. In an embodiment of the present invention, the second DCP value of each of channels is 20.

S2: Determine whether an impedance measurement command is received; and perform a next step if the measurement command is received.

S3: Switch a channel RF control module from an RF mode to a measurement mode, and simultaneously record a first DCP value of each channel.

When an operation mode of the channel RF control module is switched, a DCP value (i.e., the first DCP value) of each of the channels in the RF mode needs to be recorded and saved, and after measurement is ended, when the operation mode of the channel RF control module is switched to the RF mode again, each of the channels needs to be restored to an original first DCP value.

S4: Open a measurement channel unit, and adjust the DCP value of the channel to the set second DCP value; and simultaneously close remaining channel units, and adjust DCP values of closed channels to 0.

It needs to be noted that for a number N of channel units used in this operation, first, a first channel unit is opened, and 2^{nd} to N^{th} channel units are closed; second, a second channel unit is opened, the 1^{st} channel unit is closed, and 3^{rd} to the N^{th} channel units are closed, until all channel units are cyclically measured.

S5: The measurement channel unit emits an RF signal, and obtains an analog DC voltage and an analog DC current after conversion through a circuit; and an ADC module collects the analog DC voltage Vz and the analog DC current Iz M times, converts the collected analog DC voltage and the collected analog DC current into corresponding digital signals, and sends the corresponding digital signals to a microcontroller module.

S6: The microcontroller module performs calculation according to an impedance fitting formula, to obtain an impedance value val(x, y) for a current measurement of the channel, and stores the impedance value in a buffer of a corresponding channel inside the MCU; and increments a counter value by 1.

A number of times impedance values are stored is uniformly set in a buffer of each of channels. For example, the number of times of the impedance values are stored is set to 10. The buffer of each of the channels updates a stored impedance value based on a first-in first-out principle when the number of times the impedance values are stored exceeds the set number of times.

An impedance calculation process for current measurement channel is as follows:
First, average values of a voltage and a current are separately calculated. Based on results for M times of sampling, the average voltage value Vz_avg and the average current value Iz_avg are respectively: $Vz_avg = \left(Vz1+Vz2+Vz3+……+Vzm\right) / M$ (1) $Iz_avg = \left(Iz1+Iz2+Iz3+……+Izm\right) / M$ (2)

Vz1, Vz2, Vz3, ..., and Vzm are respectively direct current voltage values obtained through M times of sampling; and Iz1, Iz2, Iz3, ..., and Izm are respectively direct current values obtained through M times of sampling.

Second, a direct impedance value x is calculated: $x = Vz_avg / Iz_avg$ (3)

Third, an impedance value val(x, y) for current measurement is calculated based on an impedance fitting formula: $\begin{matrix}val \left(x, y\right) = p 00 + \left(p10∗x\right) + \left(p01∗y\right) + \left(p20∗{x}^{2}\right) + \left(p11∗x∗y\right) + \left(p30∗{x}^{3}\right) + \\ \left(p21∗{x}^{2}∗y\right)\end{matrix}$ (4) where x is a direct impedance value for the current measurement; y is the first DCP value; and p00, p10, p01, p20, p11, p30, and p21 are all p coefficients and are obtained through polynomial fitting on a large amount of test data. When the second DCP value is 20, a value and a range of the p coefficient are shown in Table 1.

**Table 1**

| p coefficient | Value (range) |
|---|---|
| p00 | -102.7 (-104.1 to -101.4) |
| p10 | 448.6 (445.6 to 451.6) |
| p01 | 0.01481 (0.001865 to 0.02776) |
| p20 | -301.4 (-303.7 to -299.2) |
| p11 | -0.03425 (-0.05432 to -0.01419) |
| p30 | 117.7 (117.1 to 118.2) |
| p21 | 0.02582 (0.01847 to 0.03316) |

The impedance fitting formula shown in formula (4) is obtained via fitting through switching from the RF mode to the measurement mode for multiple times and massively collected current values, voltage values, and first DCP values under different second DCP values.

S7: Determine whether a count value of a counter is equal to a number N of channel units; perform step S4 to perform measurement on a next channel unit when the count value is less than N; perform a next step when the count value is equal to N.

S8: Calculate, based on a plurality of measured impedance values of the channels, a current average impedance value of each of the channels, and display the average impedance value on a screen by a display; and reset the counter.

It needs to be noted that the plurality of impedance values of the channels refer to a plurality of measurement values stored in a buffer of a corresponding channel inside an MCU.

S9: End the current impedance measurement, switch the channel RF control module from the measurement mode to the RF mode, and restore the DCP value of each of the channels to the first DCP value.

S10: Determine whether impedance measurement during the current procedure is ended; perform a next step if the measurement is ended; and perform step S2 if next measurement is to be performed; and
S11: End the impedance measurement.

The foregoing describes in detail the bioimpedance measurement method based on multi-channel RF transmission provided in the embodiment of the present invention. Based on the above, an embodiment of the present invention further provides a bioimpedance measurement system based on multi-channel RF transmission, to implement the bioimpedance measurement method. Referring to FIG. 1, the bioimpedance measurement system includes a microcontroller module, an upper computer, and at least one channel unit. Each of the channel units includes a channel RF control module, a transformer, a current transformer, a voltage transformer, a first rectifier circuit, a second rectifier circuit, a first relay, and an analog-to-digital conversion module.

The upper computer is connected to the microcontroller module, and the microcontroller module is separately connected to each of the channel units. In each of the channel units, an output terminal of the channel RF control module is connected to an input terminal of the transformer, an output terminal of the transformer is connected to an input terminal of the first relay through the current transformer and the voltage transformer, and an output terminal of the first relay is connected to an electrode in close contact with a biological tissue; and an output terminal of the current transformer and an output terminal of the voltage transformer are respectively connected to an input terminal of the analog-to-digital conversion module through the first rectifier circuit and the second rectifier circuit, an output terminal of the analog-to-digital conversion module is connected to an input terminal of the microcontroller module, a first output terminal of the microcontroller module is connected to a control terminal of the channel RF control module, and a second output terminal of the microcontroller module is connected to a control terminal of the first relay.

The microcontroller module is configured to control an operating state of each of channel units and perform calculation based on a measurement result from the channel unit, to finally obtain an average impedance value. The upper computer is in communication with the microcontroller module and is configured to supervise and control a bioimpedance measurement process. The channel unit is configured to generate and output RF energy and measure impedance of a biological tissue.

The channel RF control module in the channel unit includes a signal generator, a signal amplifier, a DCP, a driver amplifier, and a second relay. An output terminal of the signal generator is connected to an input terminal of the signal amplifier, an output terminal of the signal amplifier is connected to an input terminal of the DCP, an output terminal of the DCP is connected to an input terminal of the driver amplifier, and an output terminal of the driver amplifier is connected to the transformer in the channel unit; a first control signal terminal of the MCU is connected to a control terminal of the DCP, and a second control signal terminal of the MCU is connected to a control terminal of the second relay; and an input terminal of the second relay is connected to a power supply terminal, and an output terminal of the second relay is connected to a power supply terminal of the driver amplifier.

The signal generator is configured to generate a sine-wave RF signal of a particular frequency and amplitude; the signal amplifier is configured to amplify the RF signal; the DCP is configured to adjust the amplitude of the outputted RF signal based on an MCU control signal; the driver amplifier is configured to perform drive amplification on the RF signal; and the second relay is configured to control an on or off state of an operating power supply for the driver amplifier based on the MCU control signal.

In conclusion, compared with the existing technology, the bioimpedance measurement method based on multi-channel RF transmission provided in the present invention uses the microcontroller module to precisely control each of the channel units, so that, for measuring each of the channels, multiple times of collection are performed under the same stable RF signal. In addition, control, collection, and feedback for each of the channels are set independently of each other, thereby avoiding mutual interference between channels and implementing accurate bioimpedance measurement. In addition, because the bioimpedance measurement system uses simpler and technically mature functional modules, such as the transformer, the transformer, the analog-to-digital conversion module, and the microcontroller module, so that the bioimpedance measurement system has beneficial effects such as good stability, low implementation costs, and high reliability.

It needs to be noted that the terms "first" and "second" are used for descriptive purposes only and should not be construed as indicating or implying relative importance or implicitly indicating a number of technical features indicated. Therefore, a feature defined with "first" or "second" may explicitly or implicitly include one or more features. In the descriptions of the present invention, "a plurality of" means two or more, unless otherwise definitely and specifically defined.

The foregoing describes in detail the bioimpedance measurement method and system based on multi-channel RF transmission provided in the present invention. For a person of ordinary skill in the art, any apparent change made to the present invention without departing from the essential content of the present invention constitutes infringement of the patent right of the present invention and bears a corresponding legal responsibility.

## Claims

1. A bioimpedance measurement method based on multi-channel radio frequency (RF) transmission, comprising the following steps:
(1) setting measurement-related parameters, comprising at least a number of channel units used during a current procedure, a number of times a voltage and a current are collected, and a second digitally controlled potentiometer (DCP) value of a channel;
(2) determining whether an impedance measurement command is received; and performing a next step if the measurement command is received;
(3) switching a channel RF control module from an RF mode to a measurement mode, and simultaneously recording a first DCP value of each channel;
(4) opening a measurement channel unit, and adjusting a DCP value of the channel to the set second DCP value; and simultaneously closing remaining channel units, and adjusting DCP values of closed channels to 0;
(5) emitting, by the measurement channel unit, an RF signal, and obtaining an analog direct current (DC) voltage and an analog DC current after conversion through a circuit; and collecting, by an analog-to-digital conversion module, the analog DC voltage and the analog DC current a set number of times, converting the collected analog DC voltage and the collected analog DC current into corresponding digital signals, and sending the corresponding digital signals to a microcontroller module;
(6) performing, by the microcontroller module, calculation according to an impedance fitting formula, to obtain an impedance value for a current measurement of the channel, and storing the impedance value in a buffer of a corresponding channel inside the microcontroller module; and incrementing a counter value by 1;
(7) determining whether a count value of a counter is equal to a set number of channel units; performing step (4) to perform measurement on a next channel unit when the count value is less than the set number of channel units; and performing a next step when the count value is equal to the set number of channel units;
(8) calculating, based on a plurality of measured impedance values of the channels, a current average impedance value of each of the channels, and displaying the average impedance value on a screen by a display; and resetting the counter;
(9) ending the current impedance measurement, switching the channel RF control module from the measurement mode to the RF mode, and restoring the DCP value of each of the channels to the first DCP value;
(10) determining whether impedance measurement during the current procedure is ended; performing a next step if the measurement is ended; and performing step (2) if next measurement is to be performed; and
(11) ending the impedance measurement.

2. The bioimpedance measurement method based on multi-channel RF transmission according to claim 1, wherein in step (6), the impedance value val(x, y) for the current measurement is calculated through the following formula: $\begin{matrix}val \left(x, y\right) = p 00 + \left(p10∗x\right) + \left(p01∗y\right) + \left(p20∗{x}^{2}\right) + \left(p11∗x∗y\right) + \left(p30∗{x}^{3}\right) \\ + \left(p21∗{x}^{2}∗y\right)\end{matrix}$ wherein x is a direct impedance value for the current measurement; y is the first DCP value; and p00, p10, p01, p20, p11, p30, and p21 are all p coefficients.

3. The bioimpedance measurement method based on multi-channel RF transmission according to claim 2, wherein the direct impedance value x is calculated through the following formula: $x = Vz_avg / Iz_avg$ wherein Vz_avg is an average voltage value, and Iz_avg is an average current value.

4. The bioimpedance measurement method based on multi-channel RF transmission according to claim 2, wherein when the second DCP value is set to 20, values of the p coefficients are as follows: $p 00 = - 102.7 ; p 10 = 448.6 ; p 01 = 0.01481 ; p 20 = - 301.4 ; p 11 = - 0.03425 ; p 30 = 117.7 ;$ and p21 = 0.02582.

5. The bioimpedance measurement method based on multi-channel RF transmission according to claim 1, wherein in step (6):
setting a uniform number of times the impedance value is stored in a buffer of each of the channels inside the microcontroller module; and updating, by the buffer of each of the channels, the stored impedance values according to a first-in first-out principle when a number of times the impedance values are stored exceeds the set number of times.

6. A bioimpedance measurement system based on multi-channel RF transmission, comprising a microcontroller module, an upper computer, and at least one channel unit, wherein
the upper computer is connected to the microcontroller module, and is configured to supervise and control a bioimpedance measurement process;
the microcontroller module is connected to each of the channel units, and is configured to control an operating state of each of the channel units, perform calculation based on a measurement result from the channel unit, and finally obtain an average impedance value; and
the channel unit is configured to generate and output RF energy and measure impedance of a biological tissue.

7. The bioimpedance measurement system based on multi-channel RF transmission according to claim 6, wherein
each of the channel units comprises a channel RF control module, a transformer, a current transformer, a voltage transformer, a first rectifier circuit, a second rectifier circuit, a first relay, and an analog-to-digital conversion module;
an output terminal of the channel RF control module is connected to an input terminal of the transformer, an output terminal of the transformer is connected to an input terminal of the first relay through the current transformer and the voltage transformer, and an output terminal of the first relay is connected to an electrode in close contact with a biological tissue; and an output terminal of the current transformer and an output terminal of the voltage transformer are respectively connected to an input terminal of the analog-to-digital conversion module through the first rectifier circuit and the second rectifier circuit, an output terminal of the analog-to-digital conversion module is connected to an input terminal of the microcontroller module, a first output terminal of the microcontroller module is connected to a control terminal of the channel RF control module, and a second output terminal of the microcontroller module is connected to a control terminal of the first relay.

8. The bioimpedance measurement system based on multi-channel RF transmission according to claim 7, wherein
the channel RF control module comprises a signal generator, a signal amplifier, a digitally controlled potentiometer (DCP), a driver amplifier, and a second relay;
an output terminal of the signal generator is connected to an input terminal of the signal amplifier, an output terminal of the signal amplifier is connected to an input terminal of the DCP, an output terminal of the DCP is connected to an input terminal of the driver amplifier, and an output terminal of the driver amplifier is connected to the transformer in the channel unit;
a first control signal terminal of the microcontroller module is connected to a control terminal of the DCP, and a second control signal terminal of the microcontroller module is connected to a control terminal of the second relay; and an input terminal of the second relay is connected to a power supply terminal, and an output terminal of the second relay is connected to a power supply terminal of the driver amplifier.
